# EUROPEAN PATENT SPECIFICATION

(11) **EP 3 848 075 B1**
(45) Date of publication and mention of the grant of the patent: **31.05.2023**
(21) Application number: 20150947.8
(22) Date of filing: 09.01.2020
(51) Int. Cl.: A61M 11/06, A61H 35/04, B05B 7/00, A61B 1/05, A61H 33/00, B05B 7/24, G06K 9/62

(54) **NASAL WASHING ASSEMBLY FOR CLEANING THE NASAL CAVITIES OF A PAEDIATRIC USER**
NASENWASCHVORRICHTUNG ZUR REINIGUNG DER NASENHÖHLEN EINES KINDES
ENSEMBLE DE LAVAGE NASAL POUR NETTOYER LES CAVITÉS NASALES D'UN ENFANT

(43) Date of publication of application: 14.07.2021
(73) Proprietor: Air Liquide Medical Systems S.R.L., 20158 Milano (IT)
(72) Inventor: ALBERICI, Luca, 25073 Bovezzo (IT); RUOCCO, Alessandra, 25073 Bovezzo (IT)
(74) Representative: Air Liquide

(56) References cited:
- WO-A1-2011/063591
- CN-A- 102 935 034
- CN-A- 107 622 284
- CN-U- 201 978 135
- CN-Y- 2 894 685
- FR-A1- 3 021 544
- US-A1- 2008 078 381

## Description

The present invention concerns a nasal washing, i.e. cleaning, assembly comprising a nasal washing device for delivering a solution, i.e. a washing/cleaning solution, such as saline solutions or the like, to the nasal cavities and/or paranasal sinuses of a paediatric user or person, i.e. baby, toddler, infant or the like, cooperating with an electronic device comprising an embedded camera, such as a smartphone or a digital pad.

Nasal washing devices, such as nebulizers, are used for delivering a nebulized solution, such as a saline solution or the like, to the nasal cavities of a person. Examples of nasal washing devices are disclosed by EP-A-2274033 and EP-A-2732881, CN 107 622 284 A, FR 3 021 544 A1, CN 102 935 034 A, CN 201 978 135 U or CN 2 894 685 Y.

The nebulized solution contributes to the removal of excessive mucus, i.e. catarrh, present in the nasal cavities and/or paranasal sinuses of the person, thereby preparing the nasal cavities and/or paranasal sinuses for subsequent administrations of one or several therapeutic agents, drugs or the like.

Without such a "pre"-washing or "pre-"cleaning, the drugs or therapeutic agents that are subsequently intra-nasally administrated, cannot act properly as they are stopped by the mucus and hence unable to reach the nasal areas, where they are supposed to act and/or to enter into the blood circulation of the person.

Nasal washing devices can be used with persons suffering from various diseases, such as rhinopharyngitis, allergic rhinitis or chronic sinusitis, and subsequently treated by anti-histaminic agents, antibiotics or decongestants that are intra-nasally delivered.

Nasal washing/cleaing is very efficient if the person accepts to have his/her nostrils cleaned by a cleaning solution, such as a saline solution.

However, this is rarely the case for a specific population of persons, namely pediatric ones, such as children, toddlers or the like. Indeed, a lot of pediatric persons are either scared by nasal washing devices and/or unable to stay calm while a nasal washing device is applied on their face, and, from time to time, even totally refuse to submit to a nasal cleaning.

It is an objective of the present invention to try to overcome the above problem in improving the acceptance of nasal washing devices by pediatric persons.

A solution according to the present invention is a nasal washing assembly, according to claim 1, comprising :
- a nasal washing device having a specific shape, and
- an electronic device comprising an embedded camera and a display screen configured for :
   ∘ recognizing in real-time the overall shape of the nasal washing device by means of the embedded camera, and
   ∘ running a predefined software in response of said real-time recognition, for displaying a predetermined video content on the display screen.

In other words, a predetermined video content is automatically and immediately displayed on the display screen of the electronic device, e.g. a smartphone, as soon as the overall shape of the nasal washing device is recognized in real-time by the electronic device, such as a microprocessor or the like of the electronic device.

It should emphasized that, in the frame of the present invention, recognizing the overall shape of the nasal washing device is faster and more convenient for the user than recognizing a tag, a bare-code, a pattern, a logo or the like, e.g. curves, waves, dots or other forms, that might be located on the main body for instance of the nasal washing device as the nasal washing device is immediately recognized without need of specific orientation and/or of minimum distance for allowing an efficient capture of the tag or the like. Further, it is also a manufacturing improvement as no tag or the like has to be arranged on the main body of the nasal washing device.

The nasal washing assembly according to the present invention as defined in claim 1, also comprises the features:
- the electronic device is configured for recognizing in real-time the overall shape of the nasal washing device, said real-time recognition comprising :
   ∘ capturing at least one picture of the overall shape of the nasal washing device by means of the embedded camera,
   ∘ processing said at least one captured picture for recognizing the overall shape of the nasal washing device, and
   ∘ running a predefined software when the overall shape of the nasal washing device is recognized, for displaying said predetermined video content on the display screen.
- processing the at least one captured picture comprises comparing said at least one captured picture with a memorized reference pattern, i.e. a memorized given shape or the like.
- Additional features are defined in the dependent claims, as follows: the predefined software is run, when said at least one captured picture matches the memorized reference pattern.
- the electronic device comprises at least one microprocessor configured for executing said real-time recognition, said captured picture processing and/or said predefined software running.
- said at least one microprocessor is a microcontroller.
- the embedded camera is configured for automatically capturing at least one picture of the overall shape of the nasal washing device in response to the real-time recognition of the overall shape of the nasal washing device.
- the microprocessor is configured for immediately running a predefined software in response to the real-time recognition of the specific overall shape of the nasal washing device.
- the predetermined video content comprises a movie, a cartoon, a video, an augmented-reality video and/or game, one or several pictures, a web page, a tutorial or the like.
- the predefined software is for instance an app, for instance an app available on Google Play Store, Apple Store or similar, i.e. a downloadable app.
- in an embodiment, the predefined software is a video reading software/app.
- in another embodiment, the predefined software is a augmented-reality software.
- in another embodiment, the predefined software is a photo editor.
- in another embodiment, the predefined software is an entertainment game or the like.
- the electronic device comprises at least one data storage memory for storing at least one memorized reference pattern and/or one or several predetermined video contents.
- the data storage memory can be a flash memory or the like.
- the electronic device comprises a smartphone or a digital pad or the like.
- the nasal washing device has an ellongated shape.
- the nasal washing device has an ellongated shape comprising a generally-cylindrical or quasi-cylindrical portion or section, and a tapered portion or section arranged above the generally-cylindrical or quasi-cylindrical portion.
- the tapered portion comprises a conical or quasi-conical portion, such as a bell-shape portion.
- the tapered portion is configured for being inserted into a nostril of a person, i.e. a baby, toddler, infant or the like.
- the generally-cylindrical or quasi-cylindrical portion or section comprises a main body and a reservoir attached to the main body.
- the reservoir, i.e. tank, contains a washing liquid solution, such as a saline solution.
- the tapered portion is arranged above the reservoir.
- the tapered portion comprises an outlet, i.e. orifice, for delivering the washing solution into the nostril(s) of the person.
- the nasal washing device has a specific shape comprising :
   ∘ a main body having a barrel shape or the like,
   ∘ a reservoir attached to the main body, having a (quasi) tubular shape or the like,
   ∘ and a nasal canula, i.e. a prong, nozzle or the like, with the exit orifice, attached to the reservoir, said nasal canula having a (quasi) conical-shape or the like.
- the display screen is a colour screen, such as a touch screen.
- the nasal washing device further comprises a gas connection for fluidly connecting the nasal washing device to a source of pressurized gas, typically air.
- the gas connection is arranged on the main body.
- the nasal washing device further comprises a actuator, such as a button or the like, actuable by a user for controlling the delivery of pressurized gas and the generation of the mist of liquid solution.
- it further comprises a pressurized-gas generator, such as air.

The present invention will be explained in more details in the following illustrative description of an embodiment of a nasal washing, i.e. cleaning, assembly according to the present invention, which is made in references to the accompanying drawings among them :
- Figure 1 illustrates an embodiment of a nasal washing device for a nasal washing assembly according to the present invention, connected to pressurized-air generator,
- Figure 2 is a side view of the nasal washing device of Figure 1, and
- Figure 3 shows a nasal washing assembly according to the present invention comprising the nasal washing device of Figure 2 cooperating with a smartphone.

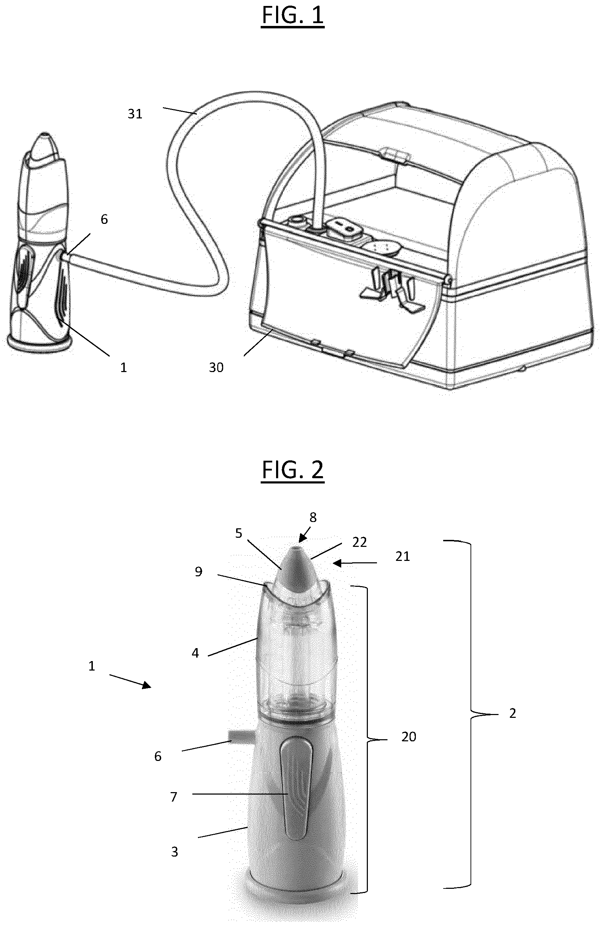

Figure 2 represent a side view of an embodiment of a nasal washing device 1 for a nasal washing assembly 1, 10 according to the present invention, that is configured for delivering a cleaning mist or aerosol into the nostrils of a person for washing the nasal cavities and/or paranasal sinuses of said person.

The mist is typically made from a liquid washing/cleaning solution, such as a saline solution or the like. It is stored into a reservoir 4 of the nasal washing assembly

The mist of washing solution, i.e. aerosol made of little dropelets of liquid, is generated into the nasal washing device 1 by an aerosol-generating system comprising for example an inner nozzle (not shown) and pressurized air for blowing the liquid solution into fine liquid particles thereby creating the desired mist of fine dropelets of liquid carried by an airflow. Aerosol-generating system are well-known.

Pressurized air is provided by means of a pressurized-gas generator 30 as shown in Figure 1, that comprises a gas compressor or the like, and that is fluidly connected, by means of a flexible hose 31 or the like, to a gas connection 6 arranged on the main body 3 of the nasal washing device 1

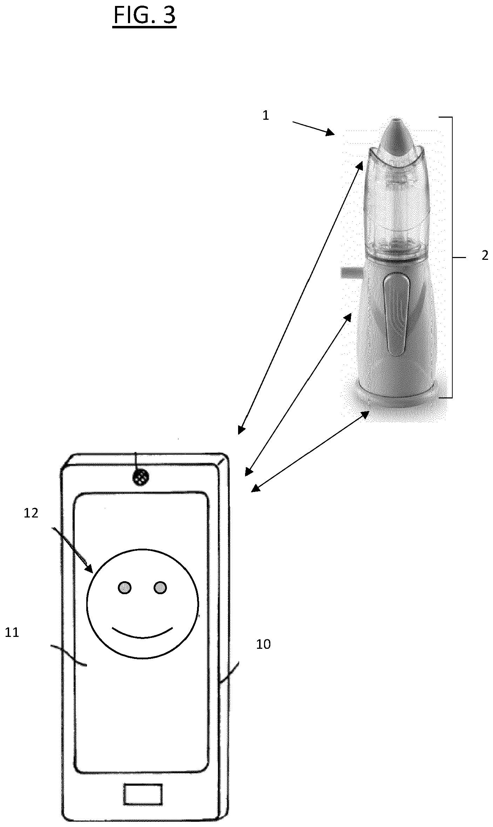

As shown in Figure 2, the nasal washing device 1 has a specific overall shape 2, i.e. outer profile, that is recognizable by a smartphone 10 of the like as explained below in reference with Figure 3.

More precisely, the nasal washing device 1 has an elongated overall shape comprising a generally-cylindrical or quasi-cylindrical portion or section 20, and a tapered portion or section 21 arranged above the generally-cylindrical or quasi-cylindrical portion 20, when the nasal washing device 1 is in its upright position as shown in Figure 2.

The tapered portion 21 comprises a conical or quasi-conical portion, such as a bell-shape portion, that comprises a nasal canula 5, i.e. nozzle, prong or the like, configured, i.e. sized, for being at least partially inserted into a nostril(s) of a person for delivering the mist of saline solution of the like therein, thereby washing the nasal cavity(ies) and/or paranasal sinus(es) of said person, i.e. a baby or the like. The mist leaves the nasal canula 5 through an outlet 8, i.e. exit orifice, arranged at the free end 22 of the nasal canula 5.

Further, the generally (quasi) cylindrical portion 20 comprises a main body 3 and a reservoir 4 attached to the main body 3, and arranged above it, whereas the tapered portion is arranged above the reservoir. The reservoir 3, i.e. tank, contains the washing liquid solution, such as a saline solution, that is sucked out by pressurized air provided by the pressurized-gas generator 30.

In the embodiment shown in Figures, the nasal washing device 1 has a specific overall shape comprising a main body 3 having a (quasi) barrel shape, a reservoir 4 having a (quasi) tubular shape, and a nasal canula 5 having a (quasi) conical-shape tapering toward the outlet 8.

Furthermore, the nasal washing device 1 also comprises a actuator 7, such as a button, that is actuable by a user for allowing pressurized gas delivered by the pressurized-gas generator 30 to travel into main body 3, through inner conduct(s) or the like, and subsequently create the desired mist in sucking some liquid solution out of the reservoir 4.

Actuator 7 cooperates with an inner valve (not shown) or the like, arranged on the air-convening conduct(s) for controlling the passage of pressurized air toward the reservoir 4 and the formation of the desired cleaning mist comprising the saline solution of the like.

The nebulized solution, i.e. mist, contributes to the removal of excessive mucus, i.e. catarrh, present in the nasal cavities and/or paranasal sinuses of the paediatric person.

The "cleaning wastes" exiting the nostrils, i.e. mixture of mucus and liquid solution, are preferably recovered by a waste-collecting chamber 9 of the nasal washing device 1, preferably the waste-collecting chamber 9 is arranged as a sleeve or skirt forming an open receptacle around the nasal canula 5, i.e. coaxially-arranged with said nasal canula 5, as shown in Figure 2. The cleaning wastes flowing out of the nostril(s) are collected by gravity and stored into said open receptacle, during the duration of the washing/cleaning.

As already explained, a lot of pediatric persons, such as babies, toddlers or young infants, are either scared by such nasal washing devices 1 and/or unable to stay calm while a nasal washing/cleaning is provided by means of such a nasal washing device 1, and in some cases, even totally refuse that such a nasal cleaning is done.

Hence, in the aim of improving the acceptance of a nasal washing/cleaning 1 by a pediatric person or the like, it is proposed, according to the present invention, a nasal washing assembly 1, 10 comprising a nasal washing device 1 having a specific overall shape 2, for instance as detailed above, and an electronic device 10 comprising an embedded camera and a display screen 11, as shown in Figure 3.

The electronic device 10 comprises an embedded camera and a display screen 11, preferably a color screen or the like. It can be for instance a smartphone as illustrated in Figure 3, a digital pad or the like.

According to the invention, said electronic device 10 is configured for recognizing in real-time the overall shape 2 of the nasal washing device 1 by means of the embedded camera, and running a predefined software in response of said real-time recognition, for displaying a predetermined video content 12 on the display screen 11.

More precisely, in an embodiment, said electronic device 10 is configured for:
∘ automatically and real-time capturing at least one picture of the overall shape 2 of the nasal washing device 1 by means of the embedded camera,
∘ processing said at least one captured picture for recognizing the specific overall shape 2 of the nasal washing device 1, and
∘ running a predefined software when the specific overall shape 2 of the nasal washing device 1 is recognized, for live displaying a predetermined video content 12 on the display screen 11, such as a cartoon, a movie or others.

Indeed, when the user is a pediatric person, such as a baby, a toddler or a young child, it is mandatory to divert his/her attention so that he/she is distracted while a nasal washing is done, and thus better accepts of the use of the nasal washing device 1.

The embedded camera of the electronic device 10 used by a second person, for example one of the parents or a medical staff, such as a nurse or a physician, is used for capturing a (or several) picture(s) of the overall shape 2 of the nasal washing device 1 and for real-time recognizing said overall shape or profile 2 of said nasal washing device 1. The recognition is operated by a microprocessor configured for comparing the captured picture(s) with memorized pattern of the like and deducing from that comparison wheter the pictures(s) match(es) with the memorized pattern.

When it is the case, the microprocessor is configured for further immediately running a predefined software, thereby live displaying a predetermined video content on the display screen 11 of the electronic device 10, such as the color screen of a smartphone, pad or the like.

The predetermined video content can be a cartoon, a movie or the like.

The baby/child sees the video content 12 displayed on the screen 11 and his/her attention is automatically diverted so that, in many cases, he/she starts to accept the nasal cleaning delivered by the nasal washing device 1, without crying or refusing it.

In other words, according to the invention, the microprocessor of the electronic device 10, namely a smartphone or the like, runs a specific software, such as an "app", that uses some other ressources of the electronic device 10, in particular the embedded camera and the display screen 12. By means of the embedded camera, the electronic device 10 recognizes, in real-time, the overall shape 2, i.e. profile, of the nasal washing device 1, and in response, immediately displays a video content 12 on the display screen 11 of the nasal washing device 1.

In the frame of the present invention, the video content 12 is taken in its broader meaning for designating a content displayed on the screen of the electronic device 10. For instance, the video content 12 can be a memorized movie, cartoon or the like, or an augmented-reality content, such as a live picture of the baby's face, or a web page or any other video imagery, while his/her mother for instance is using the nasal washing device 1 for cleaning the baby's nostrils or sinuses. The live picture of the baby's face is "modified" by a specific augmented-reality software, in particular an image modification software, of the electronic device 10.

Preferably, the augmented-reality software or "app" does not really transform or change the face of the baby, but rather does add one or several dynamic contents, such as animals, objects, figures, environmental elements... on the video content 12, i.e. dynamic images, appearing on the screen 11 of the electronic device 10, which includes the baby's face.

Said several dynamic contents 12, also called augmented reality features, are generally put in motion, on or close to the person's face shown in the screen 11 thereby distracting the baby or the like, while the cleaning solution is delivered by the nasal washing device 1. For instance, augmented reality features can comprise funny elements, such as animal ears, nose..., so that the face of the baby/child live appears on the display 12 as a transformed face, e.g. a "funny" face, comprising funny elements superimposed on the face of the baby/child.

The augmented reality features start immediately and automatically when the electronic devices 10 recognizes the overall shape 2 of the nasal washing device 1, thanks to the microprocessor that processes the picture(s) of the nasal washing device 1 captured in real-time by the embedded camera.

The nasal washing assembly 1, 10 according to the present invention greatly improves the acceptace of nasal clean-ups delivered to paediatric persons.

A nasal washing assembly according to the present invention can be used for washing the nasal cavities and/or paranasal sinuses of a paediatric person, such as a baby, a toddler or an infant

## Claims

1. Nasal washing assembly (1, 10) comprising :
- a nasal washing device (1) having a specific overall shape (2), and
- an electronic device (10) comprising an embedded camera and a display screen (11) configured for :
. recognizing in real-time the overall shape (2) of the nasal washing device (1) by means of the embedded camera, by :
- capturing at least one picture of the overall shape (2) of the nasal washing device (1) by means of the embedded camera, and
- processing said at least one captured picture by comparing said at least one captured picture with a memorized reference pattern for recognizing the overall shape (2) of the nasal washing device (1), and
. running a predefined software in response of said real-time recognition, when the overall shape (2) of the nasal washing device (1) is recognized, for displaying a predetermined video content (12) on the display screen (11).

2. Nasal washing assembly according to the preceding claim, **characterized in that** the predefined software is run, when said at least one captured picture matches a memorized reference pattern.

3. Nasal washing assembly according to any one of the preceding claims, **characterized in that** the electronic device (10) comprises at least one microprocessor configured for executing said real-time recognition, said captured picture processing and/or said predefined software running.

4. Nasal washing assembly according to any one of the preceding claims, **characterized in that** the electronic device (10) comprises at least one data storage memory for storing at least one memorized reference pattern and/or one or several predetermined video contents.

5. Nasal washing assembly according to any one of the preceding claims, **characterized in that** the predetermined video content comprises a movie, a cartoon, a video, an augmented-reality video and/or game, one or several pictures, a web page or a tutorial.

6. Nasal washing assembly according to any one of the preceding claims, **characterized in that** the electronic device (10) comprises a smartphone or a digital pad.

7. Nasal washing assembly according to any one of the preceding claims, **characterized in that** the nasal washing device (1) has an ellongated overall shape (2).

8. Nasal washing assembly according to any one of the preceding claims, **characterized in that** the nasal washing device (1) has an ellongated overall shape (2) comprising a generally-cylindrical or quasi-cylindrical portion (20) and a tapered portion (21) with an outlet (8) arranged above the generally-cylindrical or quasi-cylindrical portion (20).

9. Nasal washing assembly according to any one of the preceding claims, **characterized in that** the nasal washing device (1) has an ellongated tapered overall shape (2, 20, 21) comprising :
- a main body (3) having a barrel shape,
- a reservoir (4) attached to the main body (3), having a tubular shape, and
- a nasal canula (5) with outlet (8), attached to the reservoir (4), having a conical-shape.

10. Nasal washing assembly according to any one of the preceding claims, **characterized in that** the nasal washing device (1) comprises a gas connection (6) for fluidly connecting the nasal washing device to a source of pressurized gas (30), typically air.

11. Nasal washing assembly according to any one of the preceding claims, **characterized in that** the nasal washing device further comprises a actuator (7), such as a button, actuable by a user.

12. Nasal washing assembly according to any one of the preceding claims, **characterized in that** it further comprises a pressurized-gas generator (30).

13. Use of a nasal washing assembly according to any one of the preceding claims for washing the nasal cavities and/or paranasal sinuses of a paediatric person, such as a baby, a toddler or an infant.

## Patentansprüche

1. Nasenspülanordnung (1, 10), umfassend:
- eine Nasenspülvorrichtung (1) mit einer spezifischen Gesamtform (2), und
- eine elektronische Vorrichtung (10), die eine eingebettete Kamera und einen Anzeigebildschirm (11) umfasst, der konfiguriert ist zum:
. Erkennen der Gesamtform (2) der Nasenspülvorrichtung (1) in Echtzeit mittels der eingebetteten Kamera durch:
- Aufnehmen mindestens eines Bildes der Gesamtform (2) der Nasenspülvorrichtung (1) mittels der eingebetteten Kamera, und
- Verarbeiten des mindestens einen aufgenommenen Bildes durch Vergleichen des mindestens einen aufgenommenen Bildes mit einem gespeicherten Bezugsmuster zum Erkennen der Gesamtform (2) der Nasenspülvorrichtung (1), und
. Ausführen einer vordefinierten Software in Reaktion auf die Echtzeiterkennung bei Erkennen der Gesamtform (2) der Nasenspülvorrichtung (1) zum Anzeigen eines vorbestimmten Videoinhalts (12) auf dem Anzeigebildschirm (11).

2. Nasenspülvorrichtung nach dem vorhergehenden Anspruch, **dadurch gekennzeichnet, dass** die vordefinierte Software ausgeführt wird, wenn mindestens ein aufgenommenes Bild mit einem gespeicherten Bezugsmuster übereinstimmt.

3. Nasenspülvorrichtung nach einem der vorhergehenden Ansprüche, **dadurch gekennzeichnet, dass** die elektronische Vorrichtung (10) mindestens einen Mikroprozessor umfasst, der zum Ausführen der Echtzeiterkennung, dem Verarbeiten des aufgenommenen Bildes und/oder dem Ausführen der vordefinierten Software konfiguriert ist.

4. Nasenspülvorrichtung nach einem der vorhergehenden Ansprüche, **dadurch gekennzeichnet, dass** die elektronische Vorrichtung (10) mindestens einen Datenspeicher zum Speichern mindestens eines gespeicherten Bezugsmusters und/oder eines oder mehrerer vorbestimmter Videoinhalte umfasst.

5. Nasenspülvorrichtung nach einem der vorhergehenden Ansprüche, **dadurch gekennzeichnet, dass** der vorbestimmte Videoinhalt einen Film, einen Cartoon, ein Video, ein Video und/oder Spiel erweiterter Realität, ein oder mehrere Bilder, eine Webseite oder ein Tutorial umfasst.

6. Nasenspülvorrichtung nach einem der vorhergehenden Ansprüche, **dadurch gekennzeichnet, dass** die elektronische Vorrichtung (10) ein Smartphone oder ein digitales Pad umfasst.

7. Nasenspülvorrichtung nach einem der vorhergehenden Ansprüche, **dadurch gekennzeichnet, dass** die Nasenspülvorrichtung (1) eine längliche Gesamtform (2) aufweist.

8. Nasenspülvorrichtung nach einem der vorhergehenden Ansprüche, **dadurch gekennzeichnet, dass** die Nasenspülvorrichtung (1) eine längliche Gesamtform (2) aufweist, die einen im Allgemeinen zylindrischen oder quasizylindrische Abschnitt (20) und einen konischen Abschnitt (21) mit einem Auslass (8) umfasst, der über dem im Allgemeinen zylindrischen oder quasizylindrische Abschnitt (20) angeordnet ist.

9. Nasenspülvorrichtung nach einem der vorhergehenden Ansprüche, **dadurch gekennzeichnet, dass** die Nasenspülvorrichtung (1) eine längliche konische Gesamtform (2, 20, 21) aufweist, die umfasst:
- einen Hauptkörper (3) mit einer Fassform,
- einen Behälter (4), der am Hauptkörper (3) angebracht ist und eine Röhrenform aufweist, und
- eine Nasenkanüle (5) mit einem Auslass (8), die am Behälter (4) angebracht ist und eine Kegelform aufweist.

10. Nasenspülvorrichtung nach einem der vorhergehenden Ansprüche, **dadurch gekennzeichnet, dass** die Nasenspülvorrichtung (1) einen Gasanschluss (6) umfasst, um die Nasalspülvorrichtung fluidisch mit einer Quelle von Druckgas (30), typischerweise Luft, zu verbinden.

11. Nasenspülvorrichtung nach einem der vorhergehenden Ansprüche, **dadurch gekennzeichnet, dass** die Nasenspülvorrichtung (7) ferner ein Betätigungselement (7), beispielsweise einen Knopf, umfasst, das von einem Benutzer betätigt werden kann.

12. Nasenspülvorrichtung nach einem der vorhergehenden Ansprüche, **dadurch gekennzeichnet, dass** sie ferner einen Druckgasgenerator (30) umfasst.

13. Verwendung einer Nasenspülvorrichtung nach einem der vorhergehenden Ansprüche zum Spülen der Nasenhöhlen und/oder Nasennebenhöhlen eines Kindes, beispielsweise eines Babys, eines Kleinkindes oder eines Säuglings.

## Revendications

1. Ensemble de lavage nasal (1, 10) comprenant :
- un dispositif de lavage nasal (1) ayant une forme générale spécifique (2), et
- un dispositif électronique (10) comprenant une caméra intégrée et un écran d'affichage (11) configurés pour :
- reconnaître en temps réel la forme globale (2) du dispositif de lavage nasal (1) au moyen de la caméra embarquée en :
- capturant au moins une image de la forme globale (2) du dispositif de lavage nasal (1) au moyen de la caméra embarquée, et
- traiter ladite au moins une image capturée en comparant ladite au moins une image capturée avec un modèle de référence mémorisé pour reconnaître la forme globale (2) du dispositif de lavage nasal (1), et
- exécuter un logiciel prédéfini en réponse à cette reconnaissance en temps réel, lorsque la forme globale (2) du dispositif de lavage nasal (1) est reconnue, pour afficher un contenu vidéo prédéterminé (12) sur l'écran d'affichage (11).

2. Ensemble de lavage nasal selon la revendication précédente, **caractérisé en ce que** le logiciel prédéfini est exécuté lorsque ladite au moins une image capturée correspond à un modèle de référence mémorisé.

3. Ensemble de lavage nasal selon l'une quelconque des revendications précédentes, **caractérisé en ce que** le dispositif électronique (10) comprend au moins un microprocesseur configuré pour exécuter ladite reconnaissance en temps réel, ledit traitement de l'image capturée et/ou ladite exécution du logiciel prédéfini.

4. Ensemble de lavage nasal selon l'une quelconque des revendications précédentes, **caractérisé en ce que** le dispositif électronique (10) comprend au moins une mémoire de stockage de données pour stocker au moins un modèle de référence mémorisé et/ou un ou plusieurs contenus vidéo prédéterminés.

5. Ensemble de lavage nasal selon l'une quelconque des revendications précédentes, **caractérisé en ce que** le contenu vidéo prédéterminé comprend un film, un dessin animé, une vidéo, une vidéo et/ou un jeu en réalité augmentée, une ou plusieurs images, une page web ou un tutoriel.

6. Ensemble de lavage nasal selon l'une quelconque des revendications précédentes, **caractérisé en ce que** le dispositif électronique (10) comprend un smartphone ou une tablette numérique.

7. Ensemble de lavage nasal selon l'une quelconque des revendications précédentes, **caractérisé en ce que** le dispositif de lavage nasal (1) a une forme globale allongée (2).

8. Ensemble de lavage nasal selon l'une quelconque des revendications précédentes, **caractérisé en ce que** le dispositif de lavage nasal (1) a une forme globale allongée (2) comprenant une partie globalement cylindrique ou quasi-cylindrique (20) et une partie effilée (21) avec une sortie (8) disposée au-dessus de la partie globalement cylindrique ou quasi-cylindrique (20).

9. Ensemble de lavage nasal selon l'une quelconque des revendications précédentes, **caractérisé en ce que** le dispositif de lavage nasal (1) a une forme générale conique et allongée (2, 20, 21) comprenant :
- un corps principal (3) ayant une forme de tonneau,
- un réservoir (4) fixé au corps principal (3), de forme tubulaire, et
- une canule nasale (5) avec sortie (8), fixée au réservoir (4), de forme conique.

10. Ensemble de lavage nasal selon l'une quelconque des revendications précédentes, **caractérisé en ce que** le dispositif de lavage nasal (1) comprend un raccord de gaz (6) pour relier fluidiquement le dispositif de lavage nasal à une source de gaz sous pression (30), généralement de l'air.

11. Ensemble de lavage nasal selon l'une quelconque des revendications précédentes, **caractérisé en ce que** le dispositif de lavage nasal comprend en outre un actionneur (7), tel qu'un bouton, pouvant être actionné par un utilisateur.

12. Ensemble de lavage nasal selon l'une quelconque des revendications précédentes, **caractérisé en ce qu'**il comprend en outre un générateur de gaz sous pression (30).

13. Utilisation d'un ensemble de lavage nasal selon l'une quelconque des revendications précédentes pour le lavage des cavités nasales et/ou des sinus paranasaux d'un enfant, tel qu'un jeune enfant, un enfant en bas âge ou un nourrisson.
